(19) Europäisches Patentamt
European Patent Office
Office européen des brevets

(11) Publication number: **0 046 088**
**B1**

(12) # EUROPEAN PATENT SPECIFICATION

(45) Date of publication of patent specification: **30.12.86**

(51) Int. Cl.$^4$: **C 08 G 65/32,** C 08 G 18/50, C 08 G 18/32

(21) Application number: **81303660.5**

(22) Date of filing: **11.08.81**

(54) **Polyalkylenepolyamine derivatives and their use as curing agents for polyurethanes.**

(30) Priority: **12.08.80 JP 109806/80**

(43) Date of publication of application:
**17.02.82 Bulletin 82/07**

(45) Publication of the grant of the patent:
**30.12.86 Bulletin 86/52**

(84) Designated Contracting States:
**BE DE FR GB IT NL**

(56) References cited:
**DE-A-1 966 058**
**DE-A-2 721 626**
**DE-A-2 759 258**
**DE-A-2 809 977**
**DE-B-1 917 408**
**DE-B-1 966 059**
**GB-A-1 419 154**
**US-A-3 666 788**

(73) Proprietor: **TEXACO DEVELOPMENT CORPORATION**
**2000 Westchester Avenue**
**White Plains New York 10650 (US)**

(72) Inventor: **Umeda, Arihiko**
**No. 8-8, Naritahigashi, 3-Chome**
**Suginami Tokyo-To (JP)**
Inventor: **Iwase, Yoshiyuri**
**No. 4-9, Shakujiidai, 2-Chome**
**Nerimaku Tokyo-To (JP)**
Inventor: **Ota, Seiichi**
**No. 20-1, Sugano, 3-Chome**
**Ichikawa City Chiba (JP)**

(74) Representative: **Burnside, Michael et al**
**Michael Burnside & Partners 2 Serjeants' Inn**
**Fleet Street**
**London EC4Y 1HL (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 046 088 B1

**Description**

This invention relates to novel polyoxyalkylene polyamine-based curing agents for polyurethane preparation having improved reactivity toward isocyanates, and a gel time of convenient length and to their use in a process for making polyurethane, using such agents without any accelerators.

It is already known that polyoxyalkylenepolyamines can be used as curing agents when polyurethanes are manufactured from a polyisocyanate and a polyol (e.g. Japanese Official Bulletin Patent Publication 49-28914). However, polyoxyalkylenepolyamines are unsatisfactory curing agents because of their extremely high reactivity with isocyanates.

Although a method for lowering the reactivity of the amine with isocyanates by adding thereto a cyanoalkyl group or an alkyleneoxide group was proposed in Japanese Official Bulletin Patent Publication Sho. 49-28914 and US—A—4,075,130, the resulting products had insufficient solubility in polyols.

The literature on the Michael-type addition reaction feature of this invention includes:

Ogata and Asahara, Bull Chem. Soc. Japan, 39, 1486—1490 (1966);

Sanui and Ogata, Bull Chem. Soc. Japan, 1727 (1967); and

Ogata, High Polymer Chemistry, 27, 1—19, (1970).

DE—A—2721626 discloses the preparation of N,N'-polyoxyalkylene bis(pyrrolidinone-3-carboxylic acids) by reacting polyoxyalkylene polyamines with certain unsaturated dicarboxylic acids. Alkali metal salts of the cyclic products are curing agents for epoxy resins or isocyanurate foams.

DE—B—1917408, DE—A—1966058 and DE—B—1966059 describe N-hydroxyalkyl derivatives of certain polyoxyalkylene polyamines. These hydroxy-terminated products can be used as the active hydrogen component in an isocyanate polyaddition process.

None of these, however, disclose in any manner Applicants' curing agents and process for using same.

The present invention provides polyoxyalkylene-polyamine-based curing agents for making polyurethanes comprising reaction products of: (1) a polyoxyalkylenepolyamine, with (2) a derivative of acrylic acid or an alpha-substituted acrylic acid having a terminal hydroxyl group; and optionally (3) a compound having an oxirane ring, i.e. reaction products of (1) and (2) with (3), or reaction products of (1) and (3) with (2).

The invention also provides a process for making polyurethanes without using any accelerator which comprises adding to a polyol 5 to 100 percent by weight of at least of the above curing agents; condensing with an isocyanate and recovering the product so formed.

The preferred polyoxyalkylenepolyamines used in this invention include:

polyoxypropylenediamines of the formula

$$H_2NCH(CH_3)CH_2[OCH_2CH(CH_3)]_nNH_2 \qquad (I)$$

wherein n=2—50

polyoxyethylene bispropylenediamines of the formula

$$H_2N(CH_2)_3[O(CH_2)_2]_mO(CH_2)_3NH_2 \qquad (II)$$

wherein m=1—50 and

polyoxypropylenetriamines of the formula

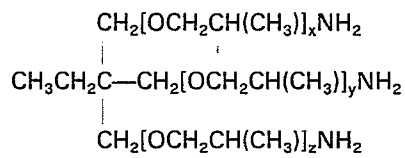

(III)

wherein x+y+z=3—10

The polyoxyalkylenepolyamine of formulae (I) and (III) are preferred. The preferred compounds of formula (I) have n=2.6, n=5.6 or n=33.1. The preferred compounds of formulae (II) and (III) are those where m=2 and x+y+z=5.3, respectively.

The preferred acrylic and alpha-substituted acrylic acid derivatives (2) are derivatives of acrylic acid, methacrylic acid and alpha-cyanoacrylic acid having the formula (IV):

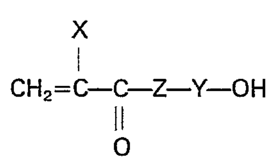

(IV)

wherein X=H, —$CH_3$, or —CN;

Y=alkylene, polyoxyalkylene or poly(alkyleneimine); and

Z=—O—, —NH— or —N—Y—OH

Exemplary compounds of formula (IV) include acrylates, methacrylates, alpha-cyanoacrylates, N-substituted acrylamides, N-substituted methacrylamides, N-substituted alpha-cyanoacrylamides, (and corresponding N,N-bis-substituted amides) having hydroxyethyl, hydroxypropyl, hydroxybutyl, polyoxyethyleneglycol, polyoxypropyleneglycol and hydroxyethyliminoethyl groups.

Of these, the preferred compounds for use in this invention are hydroxyethyl acrylate, hydroxyethyl methacrylate, hydroxyethyl cyanoacrylate and diethyleneglycol monoacrylate.

The reaction between the polyoxyalkylenepolyamine (1) and the acrylate (2) produces a Michael-type reaction adduct, as shown by I.R. spectroscopy. This reaction can be carried out with a solvent, such as an alcohol (e.g. butanol), ether (e.g. dioxane), an aromatic hydrocarbon (e.g. toluene or o-xylene) or an aliphatic hydrocarbon, e.g., in an amount equal to 10—80% of the charge reaction.

The mixture may be heated as required. The Michael adduct of the polyoxyalkylenepolyamine and the alpha-substituted acrylic acid derivative is produced almost quantitatively. Even if a small quantity of unreacted compounds is contained, it does not adversely affect polyurethane curing. Preferably the content of unreacted material should not exceed 1% of the curing agent.

Unreacted acrylate and solvent (when used) can be removed by azeotroping. To minimize the amount of unreacted acrylate in the product, an amount of acrylate close to the theoretical should be used.

The reaction temperature is generally less than 200°C and the stirring time is approx. 48 hours. Where an alphacyanoacrylic acid derivative is used as the acrylic acid derivative (2), it sometimes generates heat and therefore, cooling is necessary in such case. The molar ratio of polyoxyalkylenepolyamine (1) and acrylic acid derivative (2) is used is generally in the range of 5:1 to 1:5, preferably 3:1 to 1:3.

Preferably the oxirane compound has the formula (V)

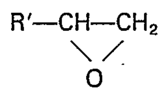

(V)

wherein R′ is hydrogen, alkyl, aryl, hydroxyalky, alkoxyalkyl or hydroxyalkaryl.

Suitable epoxy compounds include ethylene oxide, propylene oxide, n-butylglycidyl ether and styrene oxide.

The optional reaction involving the oxirane compound can comprise either initial reaction between polyoxyalkylene polyamine and oxirane compound, followed by reaction of the product with acrylate, or the reaction of the oxirane with the previously formed reaction product of acrylate and polyoxyalkylene polyamine.

A typical reaction between polyoxyalkylenepolyamine (1) and oxirane compound (2) proceeds as follows:

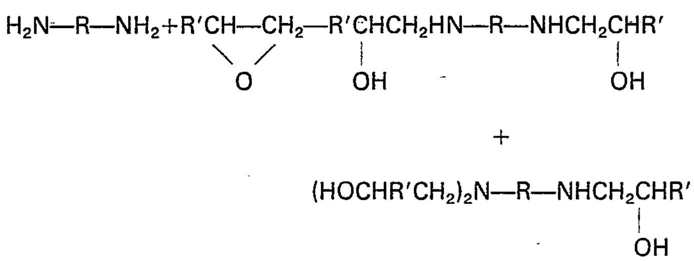

wherein

R=polyoxyalkylene, and R′ has the meaning given above.

In this reaction the preferred molar ratio of polyoxyalkylenepolyamine to oxirane compound is from 1:1.5 to 1:5.5.

The preferred parameters for the reaction of polyamine (1) with oxirane compounds (2) are as follows:

| | |
|---|---|
| a) Reactants, molar ratios; | polyamine (diamine type)/oxirane=1/1.5 to 1:3.8 mol ratio (optimum range; 1:2 to 1:3.5 mol ratio)<br>polyamine (triamine type)/oxirane=1:2 to 1:5.5 mol ratio (optimum range: 1/3 to 1:4.5 mol ratio) |
| b) Reaction temperature: | room temp. −200°C (optimum range 60—150°C) |
| c) Reaction time: | 30 minutes—48 hours (optimum range 1—20 hours) |

The subsequent reaction between (polyamine+oxirane compound) and acrylate proceeds as follows:

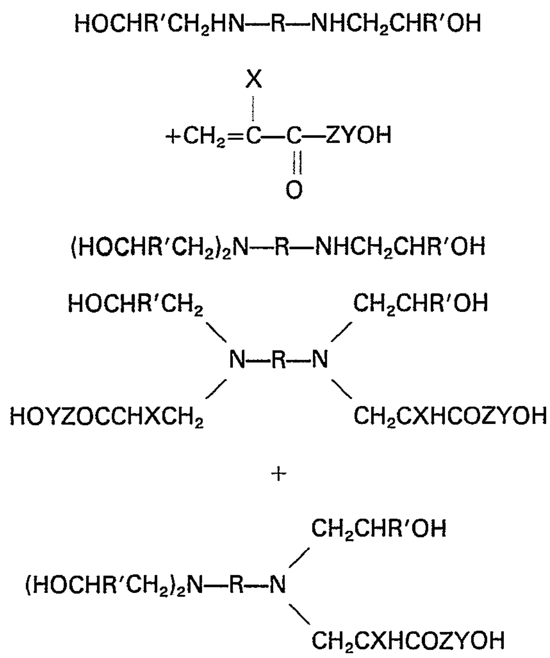

R=polyoxyalkylene; R', X, Y and Z are as above.

The preferred reaction parameters for this reaction are as follows:

| | |
|---|---|
| a) Reactants, molar ratio: | polyamine oxirane compound/acrylate=1/1 to 1:4 mol ratio (optimum range 1:2 to 1:3.5 mol ratio) |
| b) Reaction temperature: | room temp. to 200°C (optimum range: 60°C to 150°C) |
| c) Reaction time: | 1 to 48 hours (optimum range: 3 to 20 hours) |

The reaction parameters for the reaction between the polyamine (1), acrylate (2) and oxirane (3) reactants are substantially as above indicated for the polyamine, oxirane and acrylate reactants.

Unreacted acrylate and solvent (when used) can be removed by azeotroping. To minimize the amount of unreacted acrylate in the product, an amount of acrylate close to the theoretical should be used.

The invention is illustrated in non limiting fashion by the following Examples. The "reference" Examples show the use of the various curing agents in making polyurethane.

Examples 1—3

One mol of each polyoxypropylenediamine having the molecular weight shown in Table 1 was put in a 3-liter roundbottom flask. Then, 2 mols of hydroxyethylacrylate was added slowly, while stirring at temperature of 100°C. After the addition, the mixture was further stirred for 10 hours, so that a Michael addition reaction took place. The yield of adduct of polyoxypropylenediamine and hydroxyethylacrylate in each reaction product is shown in Table 1 and is nearly quantitative.

TABLE 1

| Examples | Molecular weight of polyoxypropylene-diamine | Yield of Michael adduct |
|---|---|---|
| 1 | 230 | 98% |
| 2 | 400 | 98% |
| 3 | 2000 | 95% |

Example 4

Except for the use of one mol of polyoxypropylenetriamine (400 g) having molecular weight 400 in lieu of one mol (230 g) of polyoxypropylenediamine having molecular weight of 230, and 3 mols (348 g) of hydroxyethylacrylate, the procedure of Example 1 was repeated. The yield of adduct of polyoxypropylenetriamine and hydroxyethylacrylate was 98%.

Reference Examples 1—4

0.023 mol of the compounds of Ex. 1—4 was put in a 500-ml roundbottom flask. Then, 0.05 mole of polypropyleneglycol having a molecular weight of 2,000 was added with 0.1 mol of tolylenediisocyanate. This mixture was stirred and blended at room temperature. The time required until gel of the mixture was measured as an indicator of reactivity with isocyanate. The results are shown in Table 2.

Except for using 0.023 mol of polyoxypropylenediamine having molecular weight of 230 instead of 0.023 mol of compound shown in Example 1 for comparison, the procedure was repeated, measuring the gel time.

TABLE 2

| Reference Examples | Curing agent compound | Gel time (minute) |
|---|---|---|
| 1 | Example 1 | 15 |
| 2 | Example 2 | 22 |
| 3 | Example 3 | 28 |
| 4 | Example 4 | 20 |
| Control 1 | Polyoxypropylenediamine (MW 230) | 8 seconds |

Example 5

One mole of polyoxypropylenediamine having molecular weight shown in Table 3 was put in a 3-liter round-bottom flask. Then, 2 mols (260 g) of hydroxyethylmethacrylate was dropped little by little while being stirred at temperature of 100°C. After the completion of the addition, the mixture was stirred for 10 hours, so that Michael addition reaction took place. The yield of an adduct of polyoxypropylenediamine and hydroxyethylmethacrylate in each reaction product is shown in Table 3.

TABLE 3

| Example | Molecular weight of propylenediamine | Yield of Michael adduct |
|---|---|---|
| 5 | 230 | 98% |
| 6 | 400 | 98% |
| 7 | 2000 | 98% |

Example 8

Except for using one mol (400 g) of polyoxypropylenetriamine having molecular weight of 400 instead of one mole (230 g) of polyoxypropylenediamine having molecular weight of 230, and 3 mols (390 g) of hydroxyethylmethacrylate, the procedure of Ex. 5 was repeated. The yield of an adduct of polyoxypropylenetriamine and hydroxyethylmethacrylate in the reaction product was 98%.

Reference Examples 5—8

0.02 mol of the curing agent of Examples 5—8 was put in a 500 ml round-bottom flask. Then 0.06 mol of polypropyleneglycol having a molecular weight of 2,000 was added with 0.1 mol of tolylenediisocyanate. The mixture was stirred and blended at room temperature and gel time was measured. The gel time is shown in Table 4.

Excepting for the use of 0.02 mol of polyoxypropylenetriamine (Jeffamine T-403®) in lieu of 0.02 mol of curing agent compound shown in Example 5 for comparison, the procedure of Ref. Ex. 5 was repeated. The gel time is shown in Table 4.

TABLE 4

| Reference Example | Curing agent compounds | Gel time (minute) |
|---|---|---|
| 5 | Example 5 | 17 |
| 6 | Example 6 | 23 |
| 7 | Example 7 | 28 |
| 8 | Example 8 | 21 |
| Control | Polyoxypropylenetriamine (MW 400) | 15 seconds |

Examples 9—11

One mol of polyoxypropylenediamine having molecular weight shown in Table 5 was put in a 3-liter round-bottom flask. Then 2 mols (282 g) of hydroxyethyl-alpha-cyanoacrylate was dropped little by little while being stirred at temperature of 80°C. After the completion of the addition, the mixture was stirred for 10 hours, so that a Michael addition reaction took place. The yield of an adduct of polyoxypropylenediamine and hydroxyethyl-alphacyanoacrylate is shown in Table 5.

TABLE 5

| Example | Molecular weight of polyoxypropylene-diamine | Yield of Michael adduct |
|---|---|---|
| 9 | 230 | 100% |
| 10 | 400 | 100% |
| 11 | 2000 | 100% |

Example 12

Except for the use of one mol (400 g) of polyoxypropylenetriamine instead of one mol (230 g) of polyoxypropylenediamine having molecular weight of 230, and 3 mols (423 g) of hydroxyethyl-alphacyanoacrylate ester, the procedure of Ex. 9 was repeated. The yield of an adduct of polyoxypropylenetriamine and hydroxyethyl-alphacyanoacrylate in the reaction product was 100%,

Reference Example 9—12

0.02 mol of the curing agent of Examples 9—12 was put in a 500 ml round-bottom flask. Then, 0.06 mol of polypropyleneglycol having molecular weight of 400 was added with 0.1 mol of hydrogenated diphenylmethanediisocyanate. The mixture was stirred and blended at room temperature, and gel time was measured. The gel time is shown in Table 6.

Except for the use of 0.02 mol of polyoxypropylenediamine having molecular weight of 2,000 instead of 0.02 mol of curing agent compound of Example 9 for comparison, the procedure of Ref. Ex. 9 was repeated. The gel time is shown in Table 6.

TABLE 6

| Reference Examples | Curing agent compound | Gel time (minute) |
|---|---|---|
| 9 | Example 9 | 17 |
| 10 | Example 10 | 22 |
| 11 | Example 11 | 28 |
| 12 | Example 12 | 21 |
| Control 3 | Polyoxypropylenediamine (MW 2000) | 18 seconds |

Examples 13—15

One mol of polyoxypropylenediamine having the molecular weight shown in Table 7 was put in a 3-liter round-bottom flask. Then, 2 mols (320 g) of diethyleneglycolmonoacrylate was dropped little by little

while being stirred at temperature of 100°C. After the completion of the addition, the mixture was further stirred for 10 hours, so that Michael addition reaction took place. The yield of an adduct of polyoxypropylenediamine and diethyleneglycolmonoacrylate in each reaction product is shown in Table 7.

TABLE 7

| Examples | Molecular weight of polyoxypropylene-diamine | Yield of Michael adduct |
|---|---|---|
| 13 | 230 | 98% |
| 14 | 400 | 98% |
| 15 | 2000 | 95% |

Example 16

Excepting the use of one mol (400 g) of polyoxypropylenetriamine instead of one mol (230 g) of polyoxypropylenediamine having molecular weight of 230 and 3 mols (480 g) of diethyleneglycolmonoacrylate ester, the procedure of Example 13 was repeated. The yield of an adduct of polyoxypropylenetriamine and diethyleneglycolmonoacrylate in the reaction product was 98%.

Examples 17—19

One mol of polyoxypropylenediamine having the molecular weight shown in Table 8 was put in a 3-liter round-bottom flask. Then, 2 mols (230 g) of N-hydroxyethylacrylamide was dropped little by little while being stirred at 100°C. After the completion addition, the mixture was further stirred and blended for 10 hours, so that Michael addition reaction took place. The yield of an adduct of polyoxypropylenediamine and N-hydroxyethylacrylamide for each reaction product is shown in Table 8.

TABLE 8

| Examples | Molecular weight of polyoxypropylene-diamine | Yield of Michael adduct |
|---|---|---|
| 17 | 230 | 98% |
| 18 | 400 | 98% |
| 19 | 2000 | 95% |

Example 20

Excepting the use of one mol (400 g) of polyoxypropylenetriamine having molecular weight of 400 in lieu of one mol (230 g) of polyoxypropylenediamine having molecular weight of 230, and N-hydroxyethylacrylamide (345 g), the procedure of Example 17 was repeated. The yield of an adduct of polyoxypropylenetriamine and N-hydroxyethylacrylamide in the reaction product was 98%.

Examples 21—23

One mol of polyoxypropylenediamine having molecular weight shown in Table 9 was put in a 3-liter round-bottom flask. Then, 2 mols (318 g) of N,N-bis(hydroxyethyl)acrylamide were dropped little by little while being stirred at temperature of 100°C. After the completion of the addition, the mixture was stirred for 10 hours, so that Michael addition reaction took place. The yield of an adduct of polyoxypropylenediamine and N,N-bis-(hydroxyethyl) acrylamide in each reaction product is shown in Table 9.

TABLE 9

| Examples | Molecular weight of polyoxypropylene-diamine | Yield Michael adduct |
|---|---|---|
| 21 | 230 | 98% |
| 22 | 400 | 98% |
| 23 | 2000 | 90% |

Reference Examples 6—7

The 0.02 mol of curing agent compounds shown in Examples 19 and 23 was put in a 500 ml

round-bottom flask. Then, 0.06 mol of polypropyleneglycol having molecular weight of 2,000 was added and further with 0.1 mol of tolylenediisocyanate. The mixture was stirred and blended at room temperature and gel time was measured. The results are shown in Table 10.

Except for the use of 0.02 mol of polyoxypropylenediamine having molecular weight of 2,000 in lieu of 0.02 mol of curing agent of Example 19 for comparison, the procedure of Reference Example 6 was repeated. The gel time is shown in Table 10.

TABLE 10

| Reference Examples | Curing agent compound | Gel time |
|---|---|---|
| 6 | HEAA/D 2000 (Example 19) | 35 minutes |
| 7 | BHEAA/D 2000 (Example 23) | 40 minutes |
| Control 4 | Polyoxypropylenediamine (MW 2000) | 18 seconds |

Examples 24—26

One mol of each polyoxypropylenediamine having the molecular weights shown in Table 11 was put into a 3-liter round-bottom flask. Then, 2 mols (258 g) of N-hydroxyethylmethacrylamide was added to each flask while stirring at 100°C. After completing the addition, the mixture was stirred for 10 hours and heated at 120°C, to cause a Michael addition reaction to occur. The yield of an adduct of each polyoxypropylenediamine and N-hydroxyethylmethacrylamide in each example is shown in Table 11.

TABLE 11

| Examples | Molecular weight of polyoxypropylene diamine | Yield of Michael adduct |
|---|---|---|
| 24 | 230 | 98% |
| 25 | 400 | 95% |
| 26 | 2000 | 90% |

Examples 27—29

One mol of polyoxypropylenediamine having molecular weight shown in Table 12 was put in a 3-liter round-bottom flask. Then, 2 mols (280 g) of N-hydroxyethyl-alphacyanoacrylamide was dropped little by little while being stirred at 80°C. After the completion of the addition, the mixture was further stirred for 10 hours, so that Michael addition reaction took place. The yield of an adduct of amine and N-hydroxyethyl-alphacyanoacrylamide in each reaction product is shown in Table 12.

TABLE 12

| Examples | Molecular weight of polyoxypropylene-diamine | Yield of Michael adduct |
|---|---|---|
| 27 | 230 | 100% |
| 28 | 400 | 100% |
| 29 | 2000 | 98% |

Example 30

One mol (400 g) of polyoxypropylenetriamine having molecular weight of 400 was put in a 3-liter round-bottom flask. Then, 3 mols (420 g) of N-hydroxyethyl-alphacyanoacrylamide were dropped little by little while being stirred at 80°C. After the completion of the drop, the mixture was further stirred for 10 hours, so that Michael addition reaction took place. The yield of an adduct in the reaction product was 100%.

Example 31

Using bis (aminopropyl) polyoxyethyleneglyol ether instead of polyoxypropylenediamine, the procedure of Example 9 was repeated. The yield of adduct was 98%.

Example 32—34

One mol of polyoxypropylenediamine having molecular weight shown in Table 13 was put in a 3-liter round-bottom flask. Then, it was heated at 140°C under nitrogen and 3 mols of n-butylglycidylether were slowly added. After the completion of the addition, the mixture was subjected to reaction for 2 hours. Unreacted n-butylglycidylether was evaporated under reduced pressure and a polyoxypropylenediamine n-butylglycidylether adduct was obtained.

The hydroxyl value of each reaction adduct is shown in Table 13.

TABLE 13

| Examples | Polyoxypropylene (molecular weight) | Hydroxyl value of adduct (KOH mg/g) | Code |
|---|---|---|---|
| 32 | 230 | 237 | nBGE/D-230 |
| 33 | 400 | 178 | nBGE/D-400 |
| 34 | 2000 | 54 | nBGE/D-2000 |

Example 35

Except for the use of one mol (400 g) of polyoxypropylenetriamine having molecular weight of 400 instead of one mol (2,000 g) of polyoxypropylenediamine having molecular weight of 2,000, and 4 mols (520 g) of n-butylglycidylether, the procedure of Examples 32—34 was repeated. The hydroxyl value of obtained polyoxypropylenetriamine n-butylglycidylether reaction adduct (code: nBGE/T-403) was 221 KOH mg/g.

Example 36—38

One mol (2,280 g) of the product of each of Examples 32 to 34 was put in a respective 5-liter round-bottom flask. Then, it was heated at 100°C under nitrogen and 2.5 mols of hydroxyethylacrylate was dropped little by little. After the completion of the addition, the mixture was subjected to further reaction for 5 hours. After the end of reaction, the unreacted acrylate was evaporated under reduced pressure and the acrylate adduct was obtained. The results of each reaction product are shown in Table 14.

TABLE 14

| Examples | nBGE adduct | Functionality[1] | Average molecular weight | Hydroxyl value (KOH mg/g) | Code |
|---|---|---|---|---|---|
| 36 | nBGE/D-230 | 4 | 720 | 310 | HEA/nBGE/D-230 |
| 37 | nBGE/D-400 | 4 | 890 | 250 | HEA/nBGE/D-400 |
| 38 | nBGE/D-2000 | 4 | 2500 | 90 | HEA/nBGE/D-200 |

[1] Equivalent to imino or reactive hydrogen.

Example 39

Except for the use of one mole (680 g) of "BGE/T 403 obtained in Example 35 and 3.75 mols (435 g) of hydroxyethylacrylate, the procedure of Ex. 36—38 was repeated. The obtained adduct of n-BGE/T-403 and hydroxyethylacrylate (code HEA/nBGE/T-403) has a functionality of 6, an average molecular weight of 900 and a hydroxyl value of 370 KOH mg/g.

Reference Examples 8—11

0.1 mol of the curing agent of Examples 36—39 was put in a 500-ml round-bottom flask. Then, 0.2 mol of tolylenediisocyanate was added. The mixture was stirred and blended. Time required until the mixture was geled, was measured as an indicator of reactivity with isocyanate. The results are shown in Table 15.

TABLE 15

| Reference Examples | Curing compound | Gel time (minute) |
|---|---|---|
| 8 | HEA/nBGE/D-230 (Example 36) | 15 |
| 9 | HEA/nBGE/D-400 (Example 37) | 30 |
| 10 | HEA/nBGE/D-200 (Example 38) | 35 |
| 11 | HEA/nBGE/T-403 (Example 39) | 18 |

The molar ratios forth curing compounds of Table 15 were:

HEA/nBGE/D-230 (or D-400 or D-2000)=1.84/2.16/1 (molar ratio) in reaction product

HEA/nBGE/T-403=2.76/3.24/1 (molar ratio)

Example 40

One mol (2,232 g) of Michael-type adduct of polyoxypropylenediamine having molecular weight of 2,000 and hydroxyethylacrylate obtained in Example 3 was put in a 3-liter round-bottom flask. Then, it was stirred under nitrogen and heated at 100°C with 2.5 mol (325 g) of n-butylglycidylether were dropped little by little. After the addition, the mixture was subjected to further reaction at the same temperature for 15 hours. After completion of the reaction, unreacted n-butylglycidylether was evaporated under reduced pressure, and afterward a final product was obtained. The same procedure was carried out also with Michael-type adduct of the other polyoxypropylenediamine and hydroxyethylacrylate having different molecular weight described in the above Examples.

Reference Examples 12—13

0.05 mol of the curing agent compound of Example 40 was put in a 500 ml round-bottom flask and 0.1 mol of tolylenediisocyanate was added. The mixture was stirred at room temperature and blended. The gel time was measured. The results are shown in Table 16. (No polyol is blended).

TABLE 16

| Reference Examples | Curing agent compound | Gel time (minute) |
|---|---|---|
| 12 | HEA/D-2000/n-butylglycidylether | 35 |
| 13 | (HEA/D-400/n-butylglycidylether) | (20) |

The curing agents of the invention have utility as chain extenders for flexible urethanes, urethane latices and urethane encapsulation. They give the urethane product mechanical strength and elongation properties. The agents have improved solubility in polyols and improve cross-linking properties. The curing agents of the invention are useful in the preparation in known manner of polyurethanes by the condensation reaction of a polyisocyanate and a hydroxyl-group-containing material (polyol), according to the reaction:

$$R_1NCO+R_2OH \rightarrow R_1NHCOOR_2$$

Polyols with which the curing agents of the invention can be used include for example, polyethyleneglycol having a molecular weight of 200—600, and block polymers of polyethyleneglycol and polypropyleneglycol having a molecular weight of 400—3000.

Isocyanates with which the curing agents of the invention can be used include tolylene diisocyanate, liquid diphenylmethane diisocyanate, and polymerized aromatic isocyanates.

The curing agent according to the invention is used in a ratio of 5—100 weight percent, and preferably 10 to 20 weight percent, basis polyol.

**Claims**

1. Polyoxyalkylene polyamine-based curing agents for polyurethane preparation characterized in that they are the reaction products of (1) a polyoxyalkylene polyamine, with (2) a derivative of acrylic acid or an alpha-substituted acrylic acid having a terminal hydroxyl group, and optionally a compound having an oxirane ring, this optional reaction involving either the initial reaction between the polyoxyalkylene polyamine and the oxirane compound or the reaction of the oxirane with the previously formed reaction product of acrylate and polyoxyalkylene polyamine.

2. Curing agents according to Claim 1 characterized in that said polyoxyalkylene polyamine (1) is a polyoxypropylenediamine of the formula:

$$H_2NCH(CH_3)CH_2[OCH_2CH(CH_3)]_nNH_2 \qquad (I)$$

wherein n=2—50

a polyoxyethylene bispropylenediamine of the formula:

$$H_2N(CH_2)_3[O(CH_2)_2]_mO(CH_2)_3NH_2 \qquad (II)$$

wherein, m=1—50 or a polyoxypropylene triamine of the formula:

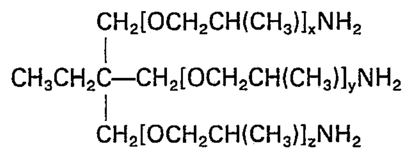

(III)

wherein x+y+z=3—10

3. Curing agents according to Claim 1 or 2 characterized in that said acrylic or substituted acrylic acid derivative (2) has the formula:

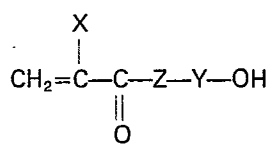

(IV)

wherein

X=H, —$CH_3$, or —CN;

Y=alkylene, polyoxyalkylene or poly(alkylene imine); and

Z=—O—, —NH— or —N—Y—OH

4. Curing agents according to any of Claims 1 to 3 characterized in that said compound having an oxirane ring (3) has the formula:

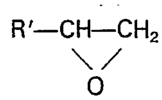

(V)

wherein R′ is hydrogen, alkyl, aryl, hydroxyalkyl, alkoxyalkyl or hydroxyalkaryl.

5. Curing agents according to any of Claims 1 to 4 characterized in that the molar ratio of polyoxyalkylene polyamine (1) to acrylic acid derivative (2) is from 5:1 to 1:5.

6. Curing agents according to any of Claims 1 to 4 characterized in that the molar ratio of reaction product of (1) and (3) with (2) is from 1:1 to 1:4.

7. Curing agents according to any of Claims 1 to 4 characterized in that the molar ratio of reaction product of (1) and (2) with (3) is from 1:1 to 1:4.

8. Curing agents according to any of Claims 1 to 7 characterized in that they also contain up to 1% of unreacted polyoxyalkylenepolyamine (1) and/or unreacted acrylic acid derivative (2).

9. The use of a curing agent according to any of Claims 1 to 8 in a process for preparing polyurethanes by condensing a polyisocyanate and a hydroxyl-containing compound, said curing agent being used in an amount of from 5 to 100 weight percent, based on said hydroxyl-containing compound.

**Patentansprüche**

1. Härter für die Polyurethan-Herstellung auf der Basis von Polyoxyalkylen-polyaminen, dadurch gekennzeichnet, daß sie die Umsetzungsprodukte sind von

(1) einem Polyoxyalkylen-polyamin mit

(2) einem Derivat der Acrylsäure oder einer alpha-substituierten Acrylsäure mit einer endständigen Hydroxylgruppe und ggf.

(3) einer Verbindung mit einem Oxiran-Ring, wobei die Gegebenenfalls-Reaktion entweder zunächst die Umsetzung des Polyoxyalkylen-polyamins mit der Oxiran-Verbindung oder die Umsetzung des Oxirans mit dem zuvor hergestellten Umsetzungsprodukt von Acrylat und Polyoxyalkylen-polyamin beinhaltet.

2. Härter nach Anspruch 1, dadurch gekennzeichnet, daß das Polyoxyalkylen-polyamin (1) ein Polyoxypropylendiamin der Formel:

$$H_2NCH(CH_3)CH_2[OCH_2CH(CH_3)]_nNH_2, \qquad (I)$$

worin n=2 bis 50,

ein Polyoxyäthylen-bispropylendiamin der Formel:

$$H_2N(CH_2)_3[O(CH_2)_2]_mO(CH_2)_3NH_2, \qquad (II)$$

worin m=1 bis 50, oder

ein Polyoxypropylen-triamin der Formel:

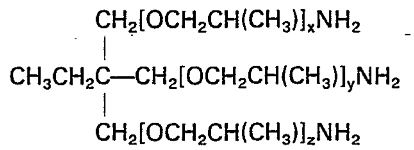

(III)

worin x+y+z=3 bis 10, ist

3. Härter nach Anspruch 1 oder Anspruch 2, dadurch gekennzeichnet, daß das Acrylsäure-oder das substituierte Acrylsäure-Derivat (2) die folgende Formel besitzt:

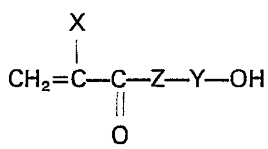

(IV)

worin X=H, —$CH_3$ oder —CN

Y=Alkylen, Polyoxyalkylen oder Poly(alkylenimin); und

Z=—O—, —NH— oder —N—Y—OG bedeuten.

4. Härter nach einem der Ansprüche 1 bis 3, dadurch gekennzeichnet, daß die Verbindung mit einem Oxiran-Ring (3) die Formel besitzt:

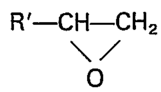

(V)

worin R' Wasserstoff, Alkyl, Aryl, Hydroxyalkyl, Alkoxyalkyl oder Hydroxyalkaryl bedeutet.

5. Härter nach einem der Ansprüche 1 bis 4, dadurch gekennzeichnet, daß das Molverhältnis von Polyoxyalkylen-polyamin (1) zum Acrylsäure-Derivat (2) 5:1 bis 1:5 beträgt.

6. Härter nach einem der Ansprüche 1 bis 4, dadurch gekennzeichnet, daß das Molverhältnis des Umsetzungsprodukts von (1) und (3) mit (2) von 1:1 bis 1:4 beträgt.

7. Härter nach einem der Ansprüche 1 bis 4, dadurch gekennzeichnet, daß das Molverhältnis des Umsetzungsprodukts von (1) und (2) mit (3) von 1:1 bis 1:4 beträgt.

8. Härter nach einem der Ansprüche 1 bis 7, dadurch gekennzeichnet, daß sie auch bis zu 1% nichtumgesetztes Polyoxyalkylen-polyamin (1) und/oder nichtumgesetztes Acrylsäure-Derivat (2) enthalten.

9. Die Anwendung eines Härters nach einem der Ansprüche 1 bis 8 in einem Verfahren zur Herstellung von Polyurethanen durch Kondensation eines Polyisocyanats und einer Hydroxyl-enthaltenden Verbindung, wobei der Härter in einer Menge von 5 bis 100 Gew.%, bezogen auf die Hydroxyl-enthaltende Verbindung, eingesetzt wird.

**Revendications**

1. Agents de durcissement à base de polyoxyalkylène polyamine pour la préparation de polyuréthanes, caractérisés en ce qu'ils sont les produits de la réaction (1) d'une polyoxyalkylène polyamine avec (2) un dérivé de l'acide acrylique ou d'un acide acrylique α-substitué ayant un groupe hydroxyle terminal, et si on le désire d'un composé ayant un cycle oxirane, cette réaction facultative faisant intervenir soit la réaction initiale entre la polyoxyalkylène polyamine et le composé oxirane, soit la réaction de l'oxirane avec le produit de réaction précédemment formé de l'acrylate et de la polyoxyalkylène polyamine.

2. Agents de durcissement suivant la revendication 1, caractérisés en ce que cette polyoxyalkylène polyamine (1) est une polyoxypropylènediamine répondant à la formule:

$$H_2NCH(CH_3)CH_2[OCH_2CH(CH_3)]_nNH_2 \qquad (I)$$

dans laquelle n=2 à 50

une polyoxyéthylène bispropylène diamine répondant à la formule:

$$H_2N(CH_2)_3[O(CH_2)_2]_mO(CH_2)_3NH_2 \qquad (II)$$

dans laquelle m=1 à 50,

ou une polyoxypropylène triamine répondant à la formule:

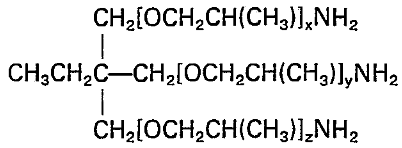

(III)

dans laquelle x+y+z=3 à 10.

3. Agents de durcissement suivant les revendications 1 ou 2, caractérisés en ce que cet acide acrylique ou ce dérivé substitué de l'acide acrylique (2) répond à la formule:

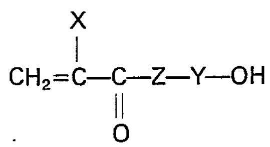

(IV)

dans laquelle X=H, $—CH_3$, ou —CN;

Y=alkylène, polyoxyalkylène ou poly(alkylène imine); et

Z=—O—, —NH— ou —N—Y—OH.

4. Agents de durcissement suivant l'une quelconque des revendications 1 à 3, caractérisés en ce que ce composé ayant un cycle oxirane (3) répond à la formule:

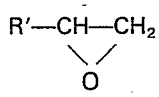

(V)

dans laquelle R' est l'hydrogène, un radical alkyle, aryle, hydroxyalkyle, alcoxyalkyle ou hydroxyalkaryle.

5. Agents de durcissement suivant l'une quelconque des revendications 1 à 4, caractérisés en ce que le rapport molaire de la polyoxyalkylène polyamine (1) au dérivé de l'acide acrylique (2) est de 5:1 à 1:5.

6. Agents de durcissement suivant l'une quelconque des revendications 1 à 4, caractérisés en ce que le rapport molaire du produit de réaction de (1) et (3) avec (2) est de 1:1 à 1:4.

7. Agents de durcissement suivant l'une quelconque des revendications 1 à 4, caractérisés en ce que le rapport molaire du produit de réaction de (1) et (2) avec (3) est de 1:1 à 1:4.

8. Agents de durcissement suivant l'une quelconque des revendications 1 à 7, caractérisés en ce qu'ils contiennent jusqu'à 1% de polyoxyalkylène polyamine (1) n'ayant pas réagi et/ou de dérivé de l'acide acrylique (2) n'ayant pas réagi.

9. Utilisation d'un agent de durcissement suivant l'une quelconque des revendications 1 à 8 dans un procédé pour préparer des polyuréthanes en condensant un polyisocyanate et un composé hydroxylé, cet agent de durcissement étant utilisé dans une quantité de 5 à 100% en poids par rapport à ce composé hydroxylé.